**Europäisches Patentamt**

**European Patent Office**

**Office européen des brevets**

(19)

(11) Publication number: **0 032 303**
A1

(12) **EUROPEAN PATENT APPLICATION**

(21) Application number: **80304637.4**

(22) Date of filing: **19.12.80**

(51) Int. Cl.³: **C 07 C 51/47**
**C 07 C 57/07**

(30) Priority: **20.12.79 US 105845**

(43) Date of publication of application:
**22.07.81 Bulletin 81 29**

(84) Designated Contracting States:
**BE CH DE FR GB IT LI LU NL SE**

(71) Applicant: **THE STANDARD OIL COMPANY**
**Midland Building**
**Cleveland, Ohio 44115(US)**

(72) Inventor: **Teng, Harry Ho I**
**36 Leonard Drive**
**Waldwick New Jersey 07463(US)**

(72) Inventor: **Bremer, Noel Jerome**
**3633 By Lane**
**Stow Ohio 44240(US)**

(72) Inventor: **Shaw, Wilfrid Garside**
**1028 Linden Avenue**
**Lyndhurst Ohio 44124(US)**

(74) Representative. Smith. Sydney et al,
**Elkington and Fife High Holborn House 52 54 High**
**Holborn**
**London WC1V 6SH(GB)**

(54) **Removal of water from aqueous carboxylic acid streams.**

(57) Water is removed from an aqueous carboxylic acid stream containing stream by the use of a polyanhydride. The aqueous carboxylic acid contains 50% by weight, preferably 80% and in particular 95% by weight of carboxylic acid. The process is particular applicable to an acid stream containing acrylic acid to product acrylic acid of high purity. The polyanhydride can be a copolymer of maleic anhydride and diisobutylene cross-linked with divinylbenzene. The polyanhydride can be regenerated for use.

EP 0 032 303 A1

TITLE

REMOVAL OF WATER FROM AQUEOUS CARBOXYLIC ACID
STREAMS

This invention relates to the removal of water
from aqueous carboxylic acid streams, in particular
where the acid is carboxylic acid is acrylic acid,
to facilitate recovery of the acid.

Acrylic acid is currently manufactured by the
vapour phase catalytic oxidation of propylene or
acrolein. The gaseous reactor effluent from this
process contains about 10-60 wt. % acrylic acid, with
water, acetic acid and various organic impurities.
This stream is then condensed or absorded to obtain
an aqueous solution of acrylic acid, acetic acid and
water.

Purification of this stream to obtain acrylic
acid has been extremely difficult. Simple fractional
distillation is not easy due to the relative
volatility of acrylic acid, acetic acid and water.

The prior art is replete with methods of
removing water from this system. U.S. Patent No.
3,816,524 describes a method of separating acrylic
acid from the aqueous solution. U.S. Patent No.
3,432,401 describes a method of using specific solvents
as entrainers to aid in azeotropic distillation.

Salts have also been used in an
attempt to concentrate the acrylic acid in this stream.
U.S. Patent No. 2,922,815 discloses that calcium chloride,
sodium sulfate, and dry metal salts such as nickel chloride
and nickel bromide have been used as drying agents
to concentrate the acrylic acid up to about 80%. U.S. Patent No. 3,846,488 discloses a method for accelerating the separation of an organic solvent phase from an aqueous phase obtained by the extraction of acrylic acid with a solvent by adding an alkali metal salt or ammonium salt in minute amounts to the aqueous solution.

Addition of solvents or entrainers has the disadvantage of requiring an additional step of separation and the use of salts has the disadvantage that large quantities are required or they are difficult to regenerate.

We have now found that polyanhydrides are very effective in concentrating acrylic acid by removing water from an aqueous acrylic acid stream and avoid the disadvantages referred to above.

The present invention provides a process for removing water from an aqueous carboxylic acid stream which comprises contacting said stream with a polyanhydride.

The invention also provides a process for removing water from an aqueous carboxylic acid stream comprising:

(a) contacting said aqueous stream with a polyanhydride to remove water therefrom and to concentrate the carboxylic acid; and

(b) separating the concentrated carboxylic acid stream from the polyanhydride.

Typical carboxylic acids useful in the present invention include acrylic acid and methacrylic acid. While the polyanhydride will remove water from an aqueous stream having any amount of carboxylic acid contained therein, it is preferred that the aqueous stream have at least 50%, preferably 80% and most preferably 95% concentration of acid. The present invention is thus extremely useful in the final purification steps for the production of a high purity acid.

The polyanhydride can be any polyanhydride known in the art such as aromatic, heterocylic or aliphatic. Preferably the polyanhydride will have a minimum solubility in the carboxylic acid.

The polyanhydride can be present in the polymer or co-polymer form. For example, maleic anhydride may be polymerized to form a useful polyanhydride. An example of another anhydride that may be polymerized is citraconic anhydride.

The polyanhydrides formed from two or more monomers to produce a co-polymer can also be used in accordance with the present invention. It is well known in the art that some anhydrides will polymerize themselves while other anhydrides will require a co-monomer to form a polyanhydride. It is preferred in the present invention that polyanhydride be a co-polymer.

Preferably the polyanhydride is a co-polymer of maleic anhydride. The co-monomer which is polymerised with maleic anhydride can be a monomer such as diisobutylene, dipropylene, styrene, indene, $\alpha$-methylstyrene, etc, of an $\alpha$-olefins such as propylene, isobutylene etc. The selection of the other monomer depends upon the size of the polyanhydride that is desired. One of the preferred monomers for polymerizing with maleic anhydride would be diisobutylene.

In order to minimize solubility of the polyanhydride with the carboxylic acid, one may cross-link the polyanhydride with a cross-linking agent. For example, the polyanhydride formed from maleic anhydride and diisobutylene will have some solubility in acrylic acid. However, when cross-linking with an agent such as divinylbenzene, a more rigid polymeric structure is formed that becomes relatively insoluble to acrylic acid, yet still is reactive to water. Care must be taken in using a cross-linking agent, however, for if a too rigid or cross-linked structure is produced, it is also possible that the water removal capabilities of the polyanhydride will be reduced. Cross-linking agents are known in the art and thus a detailed description is not necessary. However, it is preferred that cross-linking agents have two or three double bonds in one molecule. Examples of such agents are trialkylcyanurate and 3, 9-divinyl-2,4,8,10 tetraoxaspiro (5,5) undecane.

Once the polyanhydride has been produced, it may be contacted with the aqueous carboxylic acid stream by several different methods. Such methods may be by batch operation wherein the aqueous carboxylic acid and the polyanhydride are in a static condition in a vessel. The contacting process may also be continuous wherein the aqueous stream is passed over a bed of polyanhydride. In the continous process, after the polyanhydride has become saturated with water the stream can then be transferred to a second bed of fresh polyanhydride while the first bed is regenerated.

An advantage of the present invention is that water may be removed from an acid stream at relatively low temperatures. Temperatures of under 100°C can be used with the preferred temperatures being around 50°C. The exact temperature of course, depends upon the polyanhydride being used and the initial concentration of acid in the aqueous stream.

The amount of polyanhydride to be used can be estimated once the desired polyanhydride has been selected. For example, the polyanhydride formed from maleic anhydride and diisobutylene has one anhydride group per formula weight of polymer. Assuming that one formula weight of polyanhydride will react with one mole of water, and knowing the initial concentration of acrylic acid, the amount of polyanhydride necessary to remove all the water can be calculated.

The time necessary for extracting water from the aqueous stream, is of course the function of the initial concentration of the acid, and the effectiveness of the polyanhydride, and the temperature.

The following Examples illustrate the invention;-

Examples 1-2

Fifty parts each of maleic anhydride and diisobutylene is stir-mixed and refluxed under an atmosphere of nitrogen in a 2-litre 3-neck round-bottom flask. Nitrogen was used since moisture and free oxygen are harmful to the reaction components. One part divinylbenzene was dissolved in 200 parts toluene which served as a solvent. The solution was then injected continuously into the flask via a syringe pump at a flow rate of approximately 5 cc/minute. The reaction temperature was maintained at approximately 80°C. After refluxing for seven hours, the white cross-linked product obtained was blended and then washed with methanol. Finally, the product was dried at approximately 120°C overnight under vacuum.

The polyanhydride was then placed in a soxhlet extraction apparatus. The apparatus was modified such that all the reflux liquid from the condenser contacted the polyanhydride placed inside the thimble of the apparatus. The thimble was filled with approximately 7 grams of discrete polyanhydride particles. About 60 cc of concentrated crude acrylic acid was added to the soxhlet bottom pot. Hydroquinone was added to the crude acid liquid to give a concentrated of approximately 500 ppm in order to inhibit unwanted formation of acrylic acid polymer during the heated extraction process. The system was gradually evacuated and the liquid gently heated up to a temperature of 70-80°C. For Example 1, during reflux, the thimble was heated with an infrared lamp and maintained at approximately 55°C for approximately two hours. This time was extended for Example 2 to $2\frac{1}{2}$ hours. The results of these examples are shown in the Table.

- 6 -

### Example 3

Example 3 shows the polyanhydride of Example 1 being stri-mixed with the acrylic acid solution in an Erlenmeyer flask for 30 minutes. The liquid was then separated and analyzed.

### Examples 4-5

A second batch of maleic anhydride/diisobutylene polyanhydride was made as per Example 1, except the divinylbenzene was in 200 parts of benzene rather than toluene. Example 4 shows the effect of using this polyanhydride in the process of Example 1.

Example 5 shows the effect of regenerating polyanhydride. The spent polyanhydride from Example 4 was regenerated by vacuum heat treatment. After water and other volatiles were removed, the polyanhydride solid was ground up and reused. The results are shown in the Table.

### Table

Concentrating Acrylic Acid Solutions With
Maleic Anhydride/Disolbutylene Polyanhydride

| Example | Temp. °C | Pressure | Initial Wt.% | | Final Wt.% | |
|---|---|---|---|---|---|---|
| | | | AA | $H_2O$ | AA | $H_2O$ |
| 1 | 50 | Vac. | 86.3 | 12.3 | 93.8 | $<6$ |
| 2 | 75 | " | " | " | $>96.3$ | 0.4 |
| 3 | 50 | Atm. | 94.2 | 5.7 | 96.9 | $<3$ |
| 4 | 65 | Vac. | 86.3 | 12.3 | 92.4 | 1.5 |
| 5 | 75 | " | 94.2 | 5.7 | 98.1 | 0.2 |

As can be seen from above, polyanhydride is effective in removing water from an aqueous carboxylic acid stream. Further, the polyanhydride can easily be regenerated and reused without losing its desirable properties.

CLAIMS:-

1. A process for removing water from an aqueous carboxylic acid stream which comprises contacting said stream with a polyanhydride.

2. A process as claimed in claim 1 characterised in that the carboxylic acid is acrylic acid.

3. A process as claimed in claim 1 or 2 characterised in that the polyanhydride is formed from maleic anhydride.

4. A process as claimed in claim 3 characterised in that the polyanhydride is a co-polymer of maleic anhydride.

5. A process as claimed in claim 4 characterised in that the polyanhydride is a co-polymer of maleic anhydride and diisobutylene.

6. A process as claimed in claim 4 or claim 5 characterised in that the polyanhydride additionally contains a cross-linking agent.

7. A process as claimed in claim 6 characterised in that the cross-linking agent is divinylbenzene.

8. A process as claimed in any of claims 1 to 7 characterised in that the aqueous stream contains greater than 50% by weight carboxylic acid.

9. A process as claimed in any of claims 1 to 7 characterised in that the aqueous stream contains greater than 80% by weight carboxylic acid.

10. A process as claimed in any of claims 1 to 7 characterised in that the aqueous stream contains greater than 95% by weight of carboxylic acid.

11. A process as claimed in any of claims 1 to 11 characterised in that a concentrated carboxylic acid stream is obtained and this is separated from the polyanhydride.

12. A process as claimed in any of claims 1 to 11 characterised in that the polyanhydride is regenerated.

0032303

European Patent
Office

**EUROPEAN SEARCH REPORT**

Application number

EP 80 30 4637.4

## DOCUMENTS CONSIDERED TO BE RELEVANT

| Category | Citation of document with indication, where appropriate, of relevant passages | Relevant to claim |
|---|---|---|
| A | EP - A2 - 0 002 382 (STANDARD OIL) <br> * claim 1 * <br> -- | 1 |
| A | EP - A1 - 0 002 611 (STANDARD OIL) <br> * claim 1 * <br> ---- | 1 |

**CLASSIFICATION OF THE APPLICATION (Int. Cl.³)**

C 07 C 51/47

C 07 C 57/07

**TECHNICAL FIELDS SEARCHED (Int. Cl.³)**

B 01 D 53/28

C 07 C 51/47

C 07 C 57/07

**CATEGORY OF CITED DOCUMENTS**

X: particularly relevant
A: technological background
O: non-written disclosure .
P: intermediate document
T: theory or principle underlying the invention
E: conflicting application
D: document cited in the application
L: citation for other reasons

&: member of the same patent family, corresponding document

| X | The present search report has been drawn up for all claims |
|---|---|

| Place of search <br> Berlin | Date of completion of the search <br> 14-04-1981 | Examiner <br> KNAACK |
|---|---|---|

EPO Form 1503.1  06.78